Europäisches Patentamt

European Patent Office

Office européen des brevets

(19)

(11) Publication number : **0 418 323 B1**

# (12) EUROPEAN PATENT SPECIFICATION

(45) Date of publication of patent specification :
04.08.93 Bulletin 93/31

(21) Application number : 89907959.4

(22) Date of filing : 13.06.89

(86) International application number :
PCT/US89/02564

(87) International publication number :
WO 89/12385 28.12.89 Gazette 89/30

(51) Int. Cl.[5] : **A01H 1/00,** A01G 9/02,
C12N 5/00, C12M 1/16,
A01G 1/04

(54) INTEGUMENT AND METHOD FOR CULTURING AND GROWING ORGANIC MATERIAL.

(30) Priority : 14.06.88 US 207405

(43) Date of publication of application :
27.03.91 Bulletin 91/13

(45) Publication of the grant of the patent :
04.08.93 Bulletin 93/31

(84) Designated Contracting States :
AT BE CH DE FR GB IT LI LU NL SE

(56) References cited :
WO-A-88/06402
DE-A- 2 906 959
US-A- 3 941 662
US-A- 3 955 317
US-A- 4 063 383
US-A- 4 118 890
US-A- 4 311 477
US-A- 4 400 910

(56) References cited :
EXPERIMENTAL CELL RESEARCH, Vol. 84,
Issued January 1974, (San Diego, California
USA), Jensen et al., "Comparative growth
charcteristics of VERO cells on gas permeable
and conventional supports," pages 271-281,
see pages 272-274 in particular.
BIOTECHNOLOGY LETTERS, vol. 7, issued
July 1985, (Kew, England), Kybal et al., "A
device for cultivation of plant an animal cells",
pages 467-470, see page 468 in particular
Dodds et al., "Aseptic techniques", published
1982, by Dodds et al., (New York, New York,
USA), pages 10-13, see page 13 in particular

(73) Proprietor : AGRISTAR, INC.
P.O. Box 518 289 Lezak Road
Sealy, TX 77474 (US)

(72) Inventor : KERTZ, Malcolm, Glen
101 Meadow Circle Drive
Sealy, TX 77474 (US)

(74) Representative : Matthews, Graham Farrah et
al
BROOKES & MARTIN High Holborn House
52/54 High Holborn
London WC1V 6SE (GB)

EP 0 418 323 B1

## Description

RELATED APPLICATIONS

This application is a continuation-in-part of U. S. Application Serial No. 207,405 filed June 14, 1988, entitled "Integument and Method for Micropropagation and Culturing Organic Material" which is a continuation-in-part of U. S. Application Serial No. 021,408 filed March 4, 1987, entitled "Integument and Method for Micropropagation and Tissue Culturing".

FIELD OF THE INVENTION

The present invention relates to a new and improved integument and method for culturing and growing organic material. More particularly, the invention relates to a new and improved integument and method for enhancing the growth and reproduction of living organic materials such as plant and animal cells including plant and mammalian tissue, seeds, plants, animals, bacteria and other microorganisms, and for preventing contamination from occurring during such growth and reproduction.

BACKGROUND OF THE INVENTION

Cell and Tissue Culturing

Tissue culturing is the process of growing cells _in vitro_ and is used to grow both plant and animal cells. Tissue culturing techniques are commonly used in the early stages of the plant micropropagation process where it is desirable to rapidly produce plant cells. Improvements in tissue culturing techniques also have applications beyond the micropropagation of plants. Essentially the same culturing process is used to culture animal and even human tissue, such tissue being used in the fields of animal agriculture and human and veterinary medicine. Culturing of organic material other than plant and animal cells and tissue, such as bacteria, viruses and algeas, is also performed _in_ _vitro_ for both research and commercial purposes. Improvements in the procedures and apparatus used to reproduce and maintain these organisms would be beneficial, for example, to researchers and industry who require a large or steady supply of such material.

Seed Germination

Seeds are normally germinated in soil. The soil, however, includes many biological contaminants which can contaminate the seed. Further, the seeds are exposed to the environment which if not conducive to growth, will cause the seed not to germinate or for the new plants produced by the seed to die. Also, without a controlled ambient environment, growth of the new plants may be restricted.

Fungal and Bacterial Production

The growth, isolation and storage of bacterial and fungal cultures typically takes place in a rigid glass or plastic container. Because such containers provide limited gas exchange, such containers are inadequate.

Insects and Insect Cultures

The culturing of insects has previously taken place using glass or rigid plastic containers. Such containers are inadequate because they break and limit gas exchange.

Mushroom Production

Mushroom production is a multi-step process and varies with the type of mushrooms being produced. For example, there are six primary steps in the production of the common table or _Agaricus bisporus_ mushroom, namely (1) compost formation, (2) compost pasteurization, (3) spawning, (4) casing, (5) pinning, and (6) cropping. See P. Stamets, J. S. Chilton, _The Mushroom Cultivator_, Agarikon Press, Olympia, Washington (1983), for a general description of the mushroom production process.

With conventional technology, spawn production is carried out in stoppered plastic or glass bottles. Glass bottles are reused in spawn production because of their expense. If grown in glass bottles, the spawn must be repackaged into non-breakable containers, such as plastic or paper bags, prior to shipment because of the chance of breakage during shipment which could leave hazardous broken glass in the spawn.

Another type of container is a bag with a porous membrane filter over one portion of the bag. The porous filter allows gas exchange but also permits the entry of contaminants. Further, if spawn is grown in the filter bags, the spawn around the filter becomes desiccated due to moisture loss through the filter, is subject to microspore contamination, and does not grow uniformly. The spawn also tends to differentiate in growth character adjacent the filter area.

Conventional Growing Containers

Prior art culturing apparatus and processes have several deficiencies. One of the primary problems is contamination. Any of a wide variety of microorganisms, including viruses, bacteria, fungus, molds, yeast and single cell algae, can fill the living organic material during any of the various stages of growth and development. The smallest of these biological contaminants are the viruses, the largest are the single cell algae. A virus typically ranges in size from 0.1 to 0.45 micrometers although it is suspected that portions of the virus which are as small as 0.01 micrometers may separate from the virus and alone cause contamination. Bacteria typically range in size from 5 to 100 micrometers, while fungi and molds are usually larger than 100 micrometers. Yeast is larger than bacteria, with single cell algae, the largest of these biological contaminants, being larger than yeast.

The prior art sterilized glass or plastic containers such as test tubes, flasks, bottles, petri dishes or filter bags utilized in conventional technology have serious drawbacks. For example, since living organic material requires both carbon dioxide and oxygen to live and grow, these containers must provide a means for gas exchange. The walls of these traditional glass and plastic containers, however, are gas impermeable and do not permit the required gaseous interchange. Thus, rubber stoppers having cotton packing or some similar filter material, loosely fitting caps, or baffled plastic caps have been employed to allow an adequate exchange of gas between the living organic material and the ambient atmosphere and environment. However, such devices restrict the amount and rate of gas exchange. Further, such caps and stoppers do not totally protect the living organic material from contamination by microorganisms such as viruses, bacteria and fungi. Thus, it has been of paramount importance that the culture room and laboratory be kept extremely clean and their atmospheres filtered. Further, precise temperature, humidity, and light conditions must be maintained in the culture room.

The original cost of the traditional glass or plastic containers; the labor and equipment cost to maintain the sterility of the containers; and the added cost of the facilities, equipment, and related conditions required to maintain a sterile growing environment, all represent major cost factors associated with the use of such containers in conventional processes.

Reference is made to US Patent No 4, 189, 868 which discloses a package combination for storing and preserving a living plant such as lettuce. A bag, formed of a plastic material, is inflated with a gaseous medium. A living system is placed within the bag in such a manner that the block absorbs moisture which condenses within the bag and makes is available to the plant.

The present invention overcomes the deficiencies of the prior art techniques of growing and culturing living organic material by having the following advantages:

(1) enhanced protection from contamination;

(2) increased growth rates;

(3) no requirement for a sterile culture room;

(4) no requirement for expensive glass containers or the incurrence of replacement costs due to breakage;

(5) no labour cost associated with cleaning and sterilizing containers for reuse;

(6) an increase in the number of living organic material in a culture;

(7) a reduction by approximately one-half the amount of nutrients required in each culture;

(8) the elimination of the requirement of strict humidity control in the culture room;

(9) an increase in the number of living organic material which can be produced in the same size culture room;

(10) a reduction in the size of the media preparation area and in the size of sterilization equipment; and

(11) an increase in the number of new living organisms which can be produced by a laboratory technician.

It will be seen that the present invention overcomes the deficiencies associated with prior art growth containers.

According to the invention there is provided a system for the culturing of organic material, comprising: a medium for growing the organic material; a polyethylene membrane forming a chamber for enclosing the organic material and completely sealing the organic material and medium within said chamber; said polyethylene membrane being gas permeable and liquid impermeable and having interstices smaller than 0.01 micrometers, thereby allowing gas exchange through said membrane for the culturing of the organic material and preventing the exposure of the organic material to viral biological contaminants.

Further according to the invention there is provided a method for growing organic material, comprising the steps of: inserting the organic material and a growth medium in the cellule of a container made of a gas permeable, liquid and contaminant impermeable membrane having interstices smaller than 0.01 micrometers; enclosing completely the organic material within the cellule; and completely sealing the organic material from viral biological contaminants in the ambient environment.

The membrane may form one or more cellules which contain the living organic material and media. The cellule is sealed so as to completely enclose and seal off the living organic material from the ambient environment. The membrane is preferably made of a high density polyethylene, and more preferably of Chevron high density polyethylene 9650. The membrane provides uniform gas exchange throughout the surface area thereof and controls the moisture vapour transmission rate to inhibit desiccation of the living organic material.

With the use of a liquid impermeable membrane the nutrients or media, typically a liquid or semi-solid which sustains the living organic material's growth while in the membrane, cannot escape and dry out. Thus, using the present invention, it is also unnecessary to maintain a precise humidity level in the culture room which would again require special and costly equipment.

A completely unexpected benefit of using such a membrane is that the growth rates of the living organic material are dramatically increased. This increase is believed to occur because oxygen and carbon dioxide are available in greater quantities to the living organic material than prior art containers which impede gas exchange.

Other objects and advantages of the invention will appear from the following description.

BRIEF DESCRIPTION OF THE DRAWINGS

For a detailed description of a preferred embodiment of the invention, reference will now be made to the accompanying drawings, wherein:

Figure 1 depicts a frontal view of the integument of the present invention;

Figure 2 depicts a partial elevation cross-sectional view of the integument of Figure 1 taken along line 2-2 as shown in Figure 1 with the material of the integument enlarged;

Figure 3 depicts a partial top view of the integument of Figure 1 with the material of the integument enlarged;

Figure 4 depicts a sample of living organic material being cultured and grown in the integument of Figure 1;

Figure 5 depicts the organic material from Stage 1 being multiplied during Stage 2 in a new integument of Figure 1;

Figure 6 depicts the growth of an individual organic material during Stage 3 in a new integument of Figure 1;

Figure 7 depicts an integument pack with individual cellules of the type shown in Figure 1;

Figure 8 depicts a perspective view of an alternative embodiment of the integument of Figures 1 and 7;

Figure 9 depicts a front elevation view of the integument of Figure 8 in the open position;

Figure 10 depicts a side elevation view of the integument of Figure 9 in the open position;

Figure 11 depicts a top view of the integument of Figure 9;

Figure 12 depicts a front elevation view of the integument of Figure 8 in the folded position;

Figure 13 depicts a top view of the integument shown in Figure 12;

Figure 14 is a schematic drawing of a process for the manufacture of the high density polyethylene used for the membrane of the invention;

Figure 15 is a schematic view of the molecular structure of various polyethylenes;

Figure 16 is a graph showing the moisture vapor transmission rate of three different Chevron polyethylenes versus film thickness;

Figure 17 is a graph of the oxygen transmission rate of the three polyethylenes in Figure 16 versus film thickness; and

Figure 18 is a graph of the carbon dioxide transmission rate of the three polyethylenes of Figures 16 and 17 versus film thickness.

DESCRIPTION OF THE PREFERRED EMBODIMENT

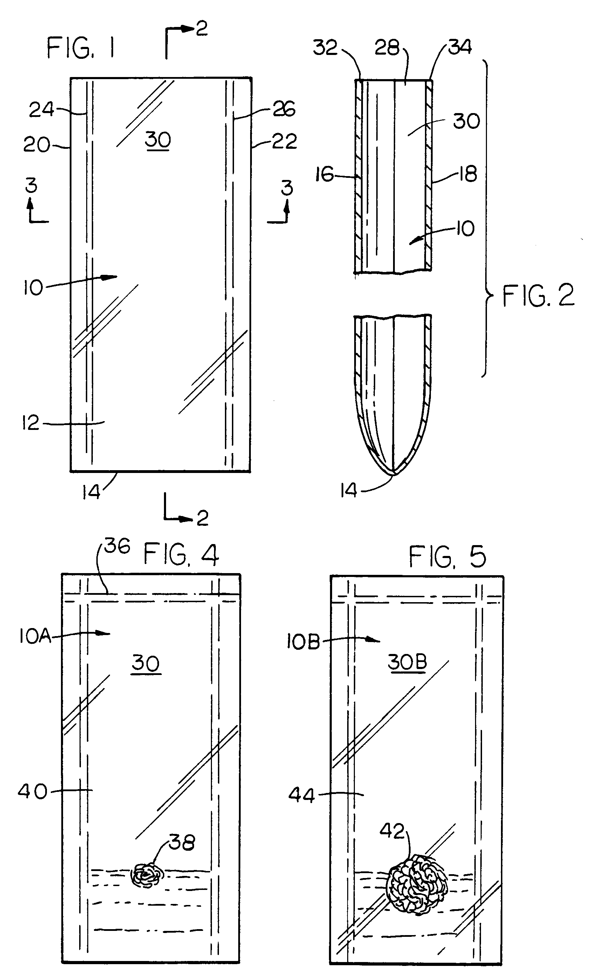

Referring initially to Figures 1, 2 and 3, there is shown the integument 10 of the present invention for containing, culturing, and growing living organic material, such as plant and animal tissue and cells, plants, seeds, spores, animals, and microorganisms including bacteria, viruses, fungus, molds and single cell algae. Integument 10 is made of a membrane 12 which is a gas permeable, liquid and contaminant impermeable material. It should be understood that the integument of the present invention may be used for culturing, growing, storing, or shipping any type of living organic material. When sealed, membrane 12 of the integument 10 completely and entirely surrounds and encloses the living organic material from the ambient environment.

The integument 10 is made by folding membrane 12 over at 14 such that two sides 16, 18 are formed. Sides 16, 18 are heat sealed at 24, 26 along the entire length thereof and adjacent to longitudinal edges 20, 22 of membrane 12 so as to form an envelope. The envelope shaped integument 10 includes a cellule 30 forming an expandable chamber for containing the living organic material and nutrients. The cellule 30 has an approximate average volume of 50 ml for most types of living organic material. As can be appreciated, the size and volume of the chamber of cellule 30 can be varied to host the particular organic material to be contained therein. Thus, cellule 30 may be of various sizes. The cellule 30 has at least initially, an open end 28 formed by the terminal edges 32, 34 of membrane 12. End 28 serves as a port of entry of cellule 30 for receiving the organic material and nutrients. As can also be appreciated, rather than being made of a single folded membrane 12, integument 10 may be made of two individual and separate pieces of material such as a base material and a frontal material. In this embodiment, the bottom of cellule 30 is formed by heat sealing the frontal material to the base material near the lower terminal edges thereof as distinguished from the fold at 14 where a single piece of material is used as described with respect to Figures 1-3. Composite integuments may be formed to take advantage of the strength of one material and the permeability to oxygen and carbon dioxide of the other, as an example. The integument 10 can also be made tubular and cut into predetermined lengths with one end closed by heat sealing.

Upon sealing and enclosing the growing living organic material within the cellule of integument 10, gases diffuse or propagate through the semipermeable membrane 12, which separates the miscible gases in the ambient atmosphere from those within the integument 10, as the gases exchange across the membrane tending to equalize their concentrations inside and outside the integument 10. The osmotic pressure or unbalanced pressure between the ambient atmosphere and the interior of the integument 10 gives rise to the diffusion and osmosis causing an interaction or interchange of gases by mutual gas penetration through the separating semipermeable membrane 12. Thus, the inventive membrane 12 of the integument 10 permits the living organic material to breathe by osmosis and air to diffuse through the semipermeable membrane 12.

The integument 10 will maintain the growth and development of living organic material therein without any artificial internal or external environment, i.e. pressures or gas concentrations. The cellule 30 need not be inflated nor must the integument 10 be placed in an incubator to create an artificial environment to maintain life within the integument 10. The gas mixture and pressure of the atmospheric ambient environment around the integument 10 tends to equalize with the gas mixture and pressure within the cellule 30 of integument 10 by gas and moisture vapor exchange through the interstices of membrane 12.

It is important for most living organic material that each wavelength of light necessary to sustain life pass through the integument. For example, individual wavelengths of light in the range of 400 to 750 nanometers are required by individual photosynthetic agents, such as the chlorophylls, to provide the reactions necessary for life and growth of plant tissue and plants. The uniformity and light cross-linking of the molecular structure of the material for membrane 12 provides a pathway of lesser resistance for light. Although the material of membrane 12 is normally translucent, the material may be opaque for the growing of mushroom spores which do not require light.

Polyethylene has been found to be the preferred material for membrane 12. Polyethylene, a thermal plastic resin, is melt formable and can be produced as a thermal plastic film. Polyethylene film is pliable and collapsible such that it can be stored and shipped in rolls. Further, polyethylene is inexpensive, as compared to glassware, such that it is disposable.

The material for membrane 12 is preferably between 1 and 2 mils thick, and it is most preferred that the material of membrane 12 have a thickness of 1.25 mils. The reduced thickness of the material for membrane 12 and the uniformity of molecular structure formed in part by the extrusion process for the material for membrane 12 enhances gas transmission. The approximate 1.25 mil thickness of the material for membrane 12 substantially enhances the amount of light and the various individual wavelengths of light which are received by the tissue culture. If the membrane material 12 is much thinner than 1.0 mil, handling the membrane 12 is made more difficult, as the opposing surfaces of the material of membrane 12 tend to adhere to each other when formed in such thin films.

The polyethylene suitable for use in the integument of the invention is described further below under the heading "Membrane Material".

Tissue Culturing and Micropropagation of Plants

The present invention may be used for the micropropagation and tissue culturing of plant tissue. Plant tissue includes any plant cell, undifferentiated plant tissue, differentiated plant tissue, pre-hardened plantlet or plant micropropagated or tissue cultured in a biological contaminant free environment during any of the stages of micropropagation or tissue culturing including initial tissue culturing, tissue culture multiplication, tissue differentiation or hardening. The term "plants" is a broader term including plant tissue as defined above and further including hardened plants which have completed the hardening stage and can grow in a natural environment where the plant is exposed to biological contaminants. "Media" is the plural for "medium". "Tissue culturing medium" generically refers to any sterile nutrient employed in the tissue culturing process including, but not limited to, minimal organic medium, multiplication medium, and pretransplant medium. The more general "medium" or "nutrient" means any nutrient for growing plants including artificial or synthetic medium, hydroponic nutrient medium, tissue culturing medium or soil.

The material for membrane 12 of integuments 10, 50 and 90 is critical to providing the desired environment for the plant tissue and plantlet during the first three stages of micropropagation and in particular enhancing growth by permitting optimum gas exchange and light transmission. Gas exchange, for example, is needed for the necessary biochemical actions required for culture growth. Understanding the role of the gases and gas exchange requires an explanation of the utilization of each gas individually.

Two functions of green plant growth are photosynthesis and respiration. Photosynthesis is the biochemical process where green plants convert carbon dioxide and water into complex carbohydrates in the presence of light of a given wave length and intensity for a given period of time. The process is affected by a number of environmental factors including quality of light, availability of water, availability of carbon dioxide, temperature, leaf age and chlorophyll content of the tissue. Photosynthesis is also referred to as a carbon dioxide fixation. The exact chemistry of the process is complex but in essence, chlorophyll in the presence of carbon dioxide, water and light converts the carbon dioxide and water into complex carbohydrates that are in turn converted into sugars and utilized by the plant as a food source.

One of the by-products of this process is the production of free oxygen. Fixation of carbon dioxide by plants accounts for a large portion of their carbon content and subsequent weight increase during growth. The exact uptake of carbon dioxide by plants varies from species to species. However, a range between eight and eighty milligrams of carbon dioxide per hour for 100 cubic centimeters of tissue surface can be used as an approximation of the carbon dioxide intake for most plants exposed to good environmental conditions. This intake can be directly related to the dry weight of plant tissue. At an uptake rate of 25 milligrams of carbon dioxide per hour for 100 cubic centimeters of tissue surface, an increase of 5% of the original weight of the tissue can be realized in a one hour period. From this overview of photosynthesis and carbon dioxide fixation, it is clear that among the critical factors affecting plant growth is the availability of carbon dioxide.

The other function relating to the gases of interest is respiration. This process is essentially an oxidation reduction reaction where oxygen serves as the oxidizer to the carbohydrates and sugars formed during the process of photosynthesis. Again, the exact chemistry involved is very complicated. However, the end result is a release of chemical energy necessary for continued growth of the plant. As in photosynthesis, or carbon dioxide fixation, a number of environmental factors affect the uptake of oxygen for the respiratory process. These include temperature, light, tissue starvation, availability of oxygen and tissue age. While respiration is believed to take place at all times in plant tissue, there is a noted increase in this activity in the absence of light. This is believed to be a result of the decreased creb cycle activity in the absence of light.

Oxygen uptake for use in respiration varies from species to species and while no generally accepted range has been established for plants in ideal environmental conditions, uptake of up to 350 microliters to 1,480 microliters per gram of fresh tissue has been recorded. There has been no direct correlation of fresh weight to oxygen uptake. There is also a difference in oxygen uptake from tissue to tissue within a given plant. Woody tissue and starch storage organs have the lowest uptake, while root tips and other regions containing meristematic cells have the highest uptake rate. This can be directly related to the activity of growth in a given area of the plant where the most active areas require the greatest energy production and consume the greatest amount of oxygen. From this, it is clearly defined that the presence of available carbon dioxide and oxygen is essential to the continued growth of green plant tissue.

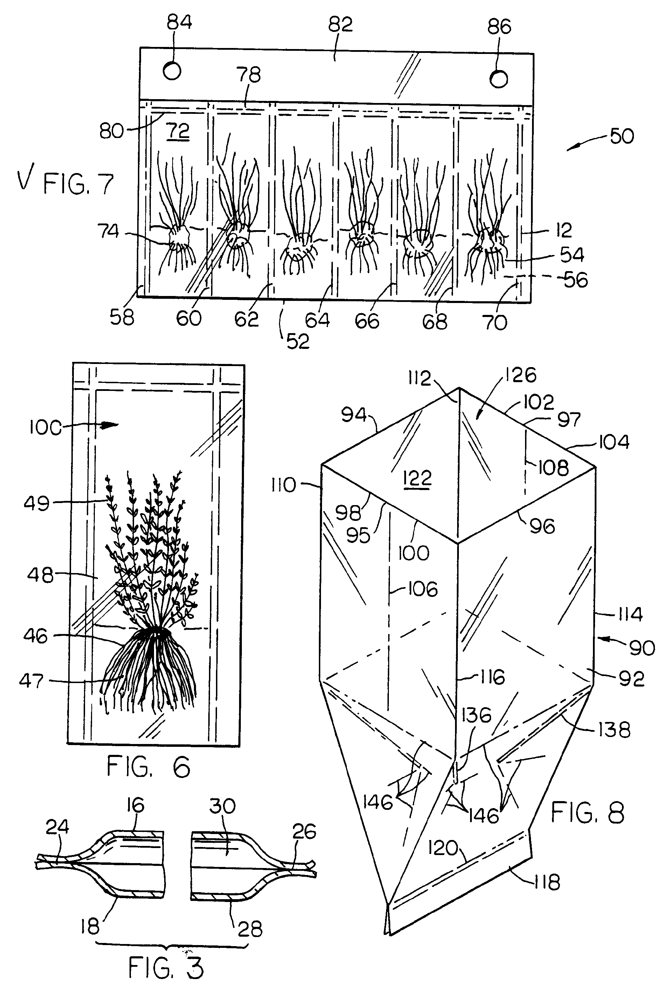

Referring now to Figures 4 to 6, the integument 10 is shown in each of the first three stages of micropropagation of plant tissue. As is shown, after the plant tissue and medium have been received by cellule 30, the port of entry at open end 28 is heat sealed at 36 along the entire length thereof and adjacent to the terminal edges 32, 34 of membrane 12 to close and seal cellule 30 containing the plant tissue and medium therein. At this time the plant tissue is completely and entirely encapsulated from the ambient environment and sealed from biological contaminants in the ambient environment. Figures 4 to 6 schematically illustrate the integuments 10A, B and C investing the plant tissue and medium in each of the first three stages of micropropagation. Figure 4 depicts the meristematic tissue 38 from a parent plant or cultivar invested within integument 10A together with suitable medium 40 such as Murashige Minimal Organic Medium manufactured by Carolina Biological Supply Company. Figure 5 illustrates the use of another integument 10B during the second stage of tissue culturing. The initial tissue culture 42 from Stage 1, or a portion of such tissue, is transferred to cellule 30B containing a suitable Stage 2 medium 44 such as Murashige Shoot Multiplication Mediums A, B and C manufactured by Carolina Biological Supply Company. Figure 6 shows an individual plantlet 46 grown in Stage 2 enclosed by another integument 10C and placed in a medium 48 such as Murashige Pretransplant Medium manufactured by Carolina Biological Supply Company to stimulate cell differentiation and the growth of individual plantlets such as 46, each plantlet 46 developing roots 47 and foliage 49.

Although the integument 10 has been shown and described as providing a single cellule 30 for enveloping an individual culture, it is preferred that the integument form a plurality of cellules. Referring now to Figure 7, there is shown an integument pack 50. Integument pack 50 is made of membrane 12 and is formed similarly to integument 10 of Figure 1. For most culture investments, the integument pack 50 has dimensions of approximately 12 inches wide and 6 inches high. Integument pack 50 is formed by folding membrane 12 over at 52 so as to form sides 54, 56. As distinguished from integument 10, sides 54, 56 are heat sealed along the entire longitudinal length thereof at 58, 60, 62, 64, 66, 68 and 70 to form six individual cellules 72. Individual tissue samples 74 and media 76 are shown invested in each of the cellules 72. The plant tissue and media may be for any of the first three stages of micropropagation as represented in Figures 4 to 6. The ports of entry at the upper end 78 have been heat sealed at 80 along the entire length of integument pack 50 to close cellules 72 after the tissue 74 and media 76 are inserted into the cellules 72. An upper flap or band 82 may be formed at the upper ends 78 of membrane 12 for the purpose of suspending integument pack 50 in the vertical position. Suitable connection means such as apertures 84, 86 may be provided through band 80 for attachment means such as drapery hooks or S-hooks to suspend integument pack 50 vertically. Suspending the growing tissue and plants vertically at different elevations markedly reduces the amount of space required in the growing areas. The suspension of cultures above others is allowed because of the translucency of integument packs 50. Further, the vertical suspension of integument packs 50 at different elevations will also enhance air movement within the growing area. Many conventional growing area layouts concentrate the tissue cultures or plants at a given elevation within the growing area, such as on countertops or working surfaces, such that there is a limited movement of air between the plants. Thus, by increasing light transmission and the availability of air for gas exchange by suspending integument packs 50, growth of the tissue and plants is enhanced and the growing area requirements are reduced.

The new and improved integument of the present invention permits the utilization of a new and improved process for micropropagation. This process includes four stages as hereinafter described.

Stage 1: Initial Tissue Culturing

The cultivars or parent plants to be micropropagated are maintained under carefully controlled greenhouse conditions in an attempt to yield plant tissue which minimizes the growth of microorganisms and particularly any biological contaminants. After selection of the optimal parent, an area of the plant with meristematic (undifferentiated) tissue is identified, and a bulk sample, which includes the meristematic tissue, is removed from the parent plant. This area is usually where active growth takes place, such as at the tips of stems or at lateral buds (between the leaf apex and the connection to the stem).

To prevent contamination of the culture by biological contaminants, the meristematic tissue is excised from the bulk sample and transferred to the growing medium under a laminar flow hood which removes airborne contaminants. Prior to the placement of the meristematic tissue sample into cellules 72 of integument pack 50, five ml of a suitable media (as distinguished from 10 ml in the prior art tissue culturing process) such as Murashige Minimal Organic Medium manufactured by Carolina Biological Supply Company is inserted into cellules 72. This medium is an agar-based substance containing all the required nutrients for tissue growth. Integument pack 50, containing the media therein, is then rolled and sterilized in an autoclave. This procedure tends to close the open upper side 78 of cellules 72. See Figure 7. Later, under the laminar flow hood, the integument packs 50 are unrolled and its cellules 72 opened one at a time prior to tissue placement. A meristematic tissue sample, typically a 0.2 to 1.0 mm cube, is then placed into an individual cellule 72 of integument pack 50, a single cellule being shown in Figure 4.

After tissue placement, the ports of entry into cellules 72 again tend to immediately close, reducing the length of time that the samples are exposed to the environment and that contaminants can enter. Thereafter, the upper ends 78 of cellules 72 are heat sealed at 80, thereby forming a complete investment and envelope around the plant tissue. In this state, the plant tissue is completely impermeable to contaminants as distinguished from the prior art containers.

The integument packs 50 are then exposed to approximately 300-500 foot-candles (3229-5382 lux) of light during this first stage.

Using the present invention process, precise temperature and humidity conditions need not be maintained in the culture room. In the prior art process, as temperature changes occurred, atmosphere would be drawn into and expelled around the tops of the glass containers containing the tissue cultures, thereby increasing the risk of contaminations from airborne contaminants which had not been removed by the prior art air filtration system. Further, the 80% humidity level was typically maintained in the prior art in order to prevent the media from drying out through evaporation. Such is not critical in the inventive process. Furthermore, and importantly, the inventive process, as distinguished from the prior art process, can be carried out in an environment which does not require a sterile, filtered air-flow since each cellule 72 of the integument pack 50 is contaminant impermeable.

Once the tissue culture has been established, and it is growing in the initial culture and has been certified contaminant-free, it is ready for Stage 2.

## Stage 2: Tissue Culture Multiplication

During Stage 2, the initial tissue culture resulting from Stage 1 is multiplied. Under the laminar flow hood, the cellules 72 of the integument packs 50 of Stage 1 are opened with a sharp sterilized knife and the tissue samples, or portions thereof, are transferred to a second set of unused integument packs 50, an individual integument pack being shown in Figure 7. Multiplication of the tissue culture occurs by using a different media. The media used for Stage 2 cultures differs from that used in Stage 1 culturing and includes hormones to induce rapid growth and multiplication of the tissue. Suitable Stage 2 media include Murashige Shoot Multiplication Mediums A, B, and C, available from the Carolina Biological Supply Company. Again, only 5 ml of media are required as compared to the 10 ml in the conventional prior art process. The integument packs 50 of Stage 2 are then heat sealed and suitably disposed on a rack within a culture room. About 300 to 500 foot-candles of light are provided. During this period, Stage 2 growth yields primarily non-differentiated tissue growth. The cells in each tissue sample multiply rapidly during Stage 2 to form a cluster of primarily undifferentiated tissue cells, the size of which depends upon the plant variety. The desired cell multiplication takes approximately 20 to 45 days, again depending upon the plant variety.

After each Stage 2 cycle, the integument packs containing the cultures are immersed in a solution of sodium hypochlorite, rinsed, returned to the laminar flow hood, opened, and the tissue is removed. The tissue is then subdivided by cutting into a number of small pieces, each of which will then be cultured. Each time the tissue samples are divided, the individual smaller tissue samples are inserted into cellules 72 of unused integument packs 50. All of these steps are performed in the laboratory under a laminar flow hood.

Each culture is grown and divided in a 20 to 45 day cycle until a sufficient number of tissue samples have been produced to meet production goals. As an example, if each tissue culture emerging from Stage 1 produces a cluster of tissue which in turn yields five tissue samples capable of culturing, over 15,000 cultures will have been produced at the end of seven months of Stage 2 multiplication. With the exception of a few naturally occurring mutations or "sports," each of these resulting cultures of Stage 2 can then be grown into an individual plant which will be genetically identical to the parent plant. Thus, when the desired number of cultures have been produced in Stage 2, the tissue cultures then are ready for Stage 3 production.

## Stage 3: Differentiation and Plant Formation

During Stage 3, the cellules 72 of the integument packs 50 of Stage 2 are opened and tissue samples therein are divided and transferred to a third set of unused integument packs 50 as shown in Figure 7. Although a single plant tissue growing into a plantlet is shown disposed within each individual cellule 72 in Figure 7, during Stage 3, a plurality of plant tissues may be disposed within an individual cellule if desired. This may be done to save additional space. However, in the inventive process, the plantlets may be grown separately in the new integument packs 50, eliminating the need for plant separation and the damage associated with untangling roots and foliage of several individual plants.

During Stage 3, the individual tissue samples grown in Stage 2 are placed in a media which stimulates cell differentiation and the growth of individual plantlets, each plantlet developing roots and foliage. Suitable Stage 3 media includes Murashige Pre-Transplant Mediums, available from the Carolina Biological Supply Company. The purpose of Stage 3 is to grow individual plantlets and prepare them for greenhouse culture. As distinguished from the prior art process, during Stage 3, the same size or a larger size integument pack 50 can be used. Initially in Stage 3, the plants are still grown in the culture room during this phase of development, but they are placed under increased light conditions so as to promote photosynthesis and growth. Approximately 2000 foot-candles of light are provided. The differentiation and growth process of Stage 3 requires between 20 and 45 days depending upon the plant variety. Because the integument packs 50 are contaminant impermeable, once individual plantlets have formed, the plantlets can be removed to the greenhouse to harden during the later portions of Stage 3 and need not be housed in a culture room for the entire Stage 3 period. This can significantly reduce the time normally required for the hardening process and reduce the size of the culture room.

Some commercial growers will purchase their plants upon completion of Stage 3. Many, however, will wait until the plants have completed Stage 4, the final production stage. If purchased at the end of Stage 3, the plants produced by the inventive process need not be immediately planted. They may be maintained for up to one month simply by keeping the plantlets in their integument packs under conditions of reasonable temperature and light. This is advantageous in commercial production where the Stage 3 plants are sometime shipped directly to the grower, who may lack the time to plant them immediately. In the prior art, since the plantlets have been removed from the sterile environment of the culture room, the commercial grower must immediately remove the plantlets from the shipping containers, rinse them to remove the media in which the contaminants can thrive, and then plant the plantlets immediately. Because the plantlets purchased by growers at the end of Stage 3 are shipped and maintained in the integument packs 50, they are contaminant impermeable and, therefore, without the danger of contamination. The advantage in the new process is that the grower does not have to plant immediately. When the grower is ready to plant, he can simply slit the Stage 3 integument open, rinse and deposit the plantlet into the soil medium.

Stage 4: Greenhouse Culture and Hardening

At Stage 4, the plantlets are removed from the integument packs 50 of Stage 3 and are transferred to a greenhouse where they are individually planted in a soil medium. The plant's tolerance to light must be increased so that the plant can adapt to its natural environment. This process is called "hardening" the plant. The plant's tolerance to light is gradually increased in Stages 3 and 4. During Stage 4, the plants are exposed to up to 8,000 foot-candles in the greenhouse where growth and hardening is to take place. The exposure of the foliage of the plant directly to the atmosphere permits the plantlet to later grow in its natural environment without the protection of the integuments used in Stages 1 to 3.

The soil used in Stage 4 is typically a pre-sterilized peat moss mix. Depending upon the type of plant, most commercial plants remain in the greenhouse 30 to 90 days before they are shipped to the grower.

Use of the inventive process permits all stages of the micropropagation process to be less time consuming than their prior art counterparts, because the new and improved integuments are more easily and quickly handled. Thus, more tissue culture samples can be processed per day. Further, because the integuments consume less space than prior art containers, the costs associated with the culture room and the greenhouse are reduced.

Examples of using the integument 10 for micropropagation and the tissue culturing are set forth in PCT International Application Number PCT/US88/00551 filed March 3, 1998, International Publication Number WO 88/06402 published September 7, 1988, incorporated herein by reference.

Micropropagation of Leafy Vegetables

Referring now to Figures 8 to 13, there is shown another embodiment of the integument of Figures 1 and 7 that is adapted and sized for the micropropagation of lettuce, spinach or other leafy vegetables. The integument 90 for enclosing the tissue for a leafy vegetable is made of the same membrane 12 as that used for integument 10 or integument pack 50 as shown in Figures 1 and 7 respectively.

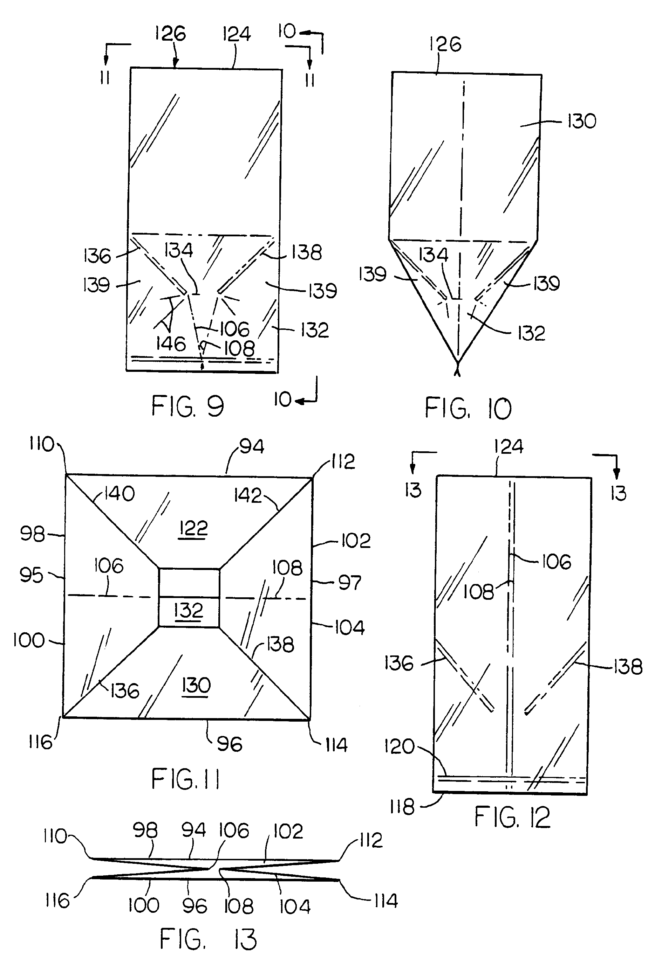

Integument 90 is extruded in tubular form having a circumference of approximately 60.96 cm (24 inches). The tubular membrane 92 is folded into quarter panels 94, 95, 96, 97 and one eighth panels 98, 100 and 102, 104, best shown in Figures 8, 11 and 13. One eighth panels 98, 100 and 102, 104 are formed by folding quarter panels 95 and 97 at 106 and 108, respectively. Quarter panels 94, 95, 96 and 97 were formed by folding tubular membrane 92 into quarter lengths, at folds 110, 112, 114, 116. Folds 106, 108 are directed inwardly as shown in Figure 13 and one end 118 of tubular membrane 92 is heat sealed at 120 in the folded position as shown in Figure 12 to produce a cellule 122 to house the leafy vegetable tissue and media. The cellule 122 has a volume of approximately 1000 ml which can be varied according to the particularly leafy vegetable plant tissue grown therein. The other end 124 of tubular membrane 92 is initially left open as a port of entry 126 to receive the leafy vegetable tissue and media.

Cellule 122 preferably includes a foliage chamber 130 and a root chamber 132 with an open neck 134 therebetween, best shown in Figure 10. Chambers 130, 132 and neck 134 are formed by heat sealing portions of one eighth panels 100 and 104 to quarter panel 96 at 136 and 138 and by heat sealing portions of one eighth panels 98 and 102 to quarter panel 94 at 140 and 142. Additionally, upon expanding integument 90, heat seals 136, 138 and 140, 142 create creases at 106, 108 and 146 shown in Figures 8 and 9 to form root chamber 132.

The foliage chamber 130 and root chamber 132 of cellule 122 permit a separation of the foliage from the root system during growth and more particularly to separate the foliage from the media. A plantlet is positioned within cellule 122 such that the foliage grows within foliage chamber 130 and the root system extends from the foliage chamber 130 down through neck 134 and into the root chamber 132 where the media is disposed. Given a cellule 122 with a volume of approximately 1000 ml, the root chamber 132 is sized to contain approximately 50 ml of media. By maintaining the integument 92 in the vertical position, all media will flow downward into root chamber 132. This downward flow is facilitated by the angular heat sealing at 136 and 138. Thus, the media is thereby kept separate from the foliage. This permits the foliage to be kept clean of media and to permit the leafy vegetable to grow in a preferred and desirable symmetric shape. Without the division of cellule 122 into a foliage and a root chamber, the leafy vegetable would grow in a haphazard form losing its symmetry. Further, with the reduced neck portion 134 separating cellule 122 into a root chamber 132 and foliage chamber 130, the media is retained in the root chamber 132 and its flow into the foliage chamber 130 is prevented or retarded when the integument 90 is tipped or inverted since the media will tend to flow into the upper angular portions 139 of root chamber 132 instead of flowing through neck position 132. Additionally, the reduced neck portion 134 tends to secure a mature plant in position within cellule 122 since the plant's roots will grow into a mass having a size larger than the cross sectional area of neck portion 134. This growth of root mass also acts to impede the flow of media into the foliage chamber 130.

Seed Germination

The integument and method of the present invention may be used to germinate seeds in a sterile environment. The following is an example of growing lettuce from seed using the integument packs 50 of the present invention.

The media, Murashige Minimal Organic, is mixed with 30 grams of sucrose and 8 grams of agar. The final pH is adjusted to 5.5. 5 ml of the media is then placed into the cellules 72 of integument packs 50 depicted in Figure 7.

Black-seeded Simpson lettuce seeds are wrapped in gauze and surface sterilized by sonicating for ten minutes in a 10% sodium hypochlorite solution to which two drops of a wetting agent had been added. The gauze packet is then removed and rinsed three times in sterile distilled water, with each rinse lasting for three minutes. The gauze packet is then placed on a sterile work surface under a laminar flow hood. The seeds are planted in the cellules 72 of integument packs 50 with one seed per cellule 72.

After each cellule 72 is filled, it is sealed, labelled and placed n the culture room where it is checked daily for growth and contamination. The culture room is maintained at 26.7°C (80°F) with sixteen hours of light and light hours of darkness per twenty-four period. The integument packs are checked daily for contamination and germination.

While this example is used the integument packs 50 such as illustrated in Figure 7, given the high germination rates of the seeds grown using the inventive process, it is preferably to grow lettuce from seed in an integument 90 such that the plantlets produced do not have to be transferred from an integument pack 50 to an integument 90. Due to the low cost of the integument 90 and the seed itself, any integument 90 containing a seed which fails to germinate can easily be identified and disposed of. Further, eliminating the steps of transferring plantlets from integument packs 50 to integuments 90 would eliminate the significant labour costs otherwise incurred.

Fungal and Bacterial Production and Culture Storage

The integument and method of the present invention may also be used for the growth, isolation and storage of bacterial and fungal cultures. For example, a fungus substrate, inoculated with an inoculant culture, can be stored, grown or transported in these integuments. Because the integument is sealed and contaminant impermeable, other molds, bacteria or fungus which could alter or damage the culture cannot enter. Another advantage of the integuments of the invention relates to the fact that fungus grows best in the presence of water. The integuments of the invention inhibit desiccation. Examples of fungal and bacterial production and culture storage are described below:

### Fungal Cultures

A semisolid Minimal Organic Media with 30 grams of sucrose and 8 grams agar was prepared. The final ph was adjusted to 5.5. 5 ml of the media was then placed into cellules 72 of integument packs 50 depicted in Figure 7. The integument packs 50 were then autoclaved for 15 minutes at 121.1°C (250°F) 10546 kg/m$^2$ at 15 psi. They were then placed in the laminar flowhood and allowed to cool. Several cellules 72 of integument packs 50 were inoculated with Rhizoctonia solani. This was done by inserting a sterile inoculation loop into a pure culture of Rhizoctonia solani, removing it and then inserting it into a cellule 72 of integument pack 50. The inoculant loop was placed in the centre of the cellule and moved down until contact was made with the media. The loop was then resterilized and the process repeated until all cellules 72 of integument pack 50 had been inoculated. The integument packs 50 were heat sealed, labelled and placed in the culture room. This process was repeated using pure cultures of Rhizopus stolonifer and Penicillium italicum on several integument packs 50.

To determine if cross contamination would occur between cellules 72 of integument pack 50, several integument packs were inoculated in the following manner. The first, third and fifth cellule 72 of the integument pack 50 was inoculated with Rhizoctonia solani, Rhizopus stolonifer and Penicillium italicum respectively, leaving cellules 2 and 4 containing only sterile media. The integument packs were sealed and each cellule containing an inoculant was labeled. The integument packs were placed in the culture room.

After 5 days all cultures were checked by visual observation for growth of fungus. All inoculated cultures showed positive growth and no growth was observed on those cellules that were not inoculated. The cultures were checked several times during a six month period. While fungal growth slowed as the media was consumed, the cultures remained viable. Little or no drying of the media was noted. Further, the resulting fungal cultures appeared to be pure, i.e., uncontaminated from outside microorganisms or from the other fungal cultures.

### Bacterial Culture

Several integument packs 50 were inoculated with the bacteria Serratia marcescens, obtained from a pure culture in the method described above for fungal cultures.

The cultures were checked by visual observation after 5 days and several times after that during a 6 month period. All inoculated cellules of the integuments showed positive growth of Serratina marcescens. It was observed for certain bacterial cultures that the bacteria migrated towards the inner surfaces of the integument membrane where the gas exchange with the ambient atmosphere was greatest. There was no bacterial growth or contamination of the cellules of the integuments that were not inoculated, and those that were inoculated appeared pure. At the end of a 6 month period all cultures were viable with little or no loss of media due to drying.

### Microorganisms

Certain microorganisms live and grow anaerobically, i.e. without oxygen. Although no such experiments have yet been conducted, the integument for culturing these microorganisms can be made from less permeable materials than the polyethylene of the preferred embodiment so as to preclude a gaseous interchange between the ambient environment and the microorganisms within the integument. Similarly, some microorganisms prefer or require the absence of light. When culturing such microorganisms, an opaque material can be used for the membrane.

### Insects and Insect Cultures

The integuments 10 of the invention can also be used for growing or storing insects or insect cultures. An insect can be placed in the integument 10 and grown. For example, butterflies from the larvae and cocoon stage can be produced in integuments. The integuments 10 of the invention retain moisture, which the insects need for growth and life, and also permit gas exchange. The integuments are also relatively low in cost and unbreakable, unlike flasks or test tubes which are conventionally used for insect storage and growth.

It should also be noted that insect cells and tissue can be cultured in the integuments 10 of the invention. The fact that the integuments 10 are contaminant impermeable, and do not allow entry of contaminants which can cause culture spoilage, is the main advantage in these latter applications.

## Growing Mushrooms

As an example of the use of the integument 10 of the present invention in the process of growing mushrooms, the process for the production of the common table or Agaricus bisporus mushroom is described below.

## Compost Formation

The production of compost for the Agaricus bisporus mushroom is initiated by mixing and wetting the ingredients. Nitrogen supplements and gypsum are preferably spread over the top of the ingredients as they are mixed. The ingredients are then removed and preferably stacked in piles. The nitrogen supplements increase nitrogen content, and the gypsum minimizes greasiness and also increases the flocculation of certain chemicals in the compost. When gypsum is added, it is generally at about 9.07 to 36.29kg (20 to 80 pounds) per tonne (ton) of dry ingredients. Organic nitrogen supplements commonly include brewer's grain, seed meals of soybeans, peanuts, or cotton, and chicken manure, among others. Other inorganic nitrogen supplements can also be added to synthetic compost, including ammonium nitrate and urea. The inorganic supplements provide microflora in the compost with a readily available source of nitrogen for their growth and reproduction.

## Compost Pasteurization

During compost pasteurization, the compost is pasteurized and the ammonia which formed during compost formation is removed. However, compost for growing oyster mushrooms would not require any nitrogen supplements, and no ammonia would be produced.

There are two major methods of compost pasteurization, namely a high temperature and a low temperature system. In both systems, the compost is packed into trays, or beds, or alternatively, simply stacked in bulk. The compost is then placed in an environmentally controlled room. It should be noted that the method of pasteurization can be varied considerably.

## Spawning

Spawn includes colonized mushroom mycelium growing in a substrate conducive to the particular type of mushroom being grown. Spawn can be produced by filling an integument 10 with the substrate, sterilizing the substrate and the integument, cooling the substrate, inoculating the substrate with mushroom culture, and colonizing the substrate to produce spawn. The spawn then can be grown in the integument or refrigerated for storage. After the substrate is colonized with mycelium, it can be grown further to produce mushrooms or, in the case of Agaricus bisporus, mixed with compost for mushroom production, as hereinafter described.

Different varieties of mushroom utilize different types of substrates to produce growth. A grain, such as rye, millet or wheat, is generally used as the substrate for spawn production of the table mushroom, Agaricus bisporus, although almost any grain may be used. Normally, in spawn production where grain is the substrate, the grain is mixed with water and chalk. The chalk maintains pH control and keeps the spawn from sticking together. Gypsum may also be added. The grain can be raw or pre-cooked. If one is producing Shiitake mushrooms, sawdust, wood plugs, small wooden dowels, or another cellulose containing material is substituted for grain as the substrate. There are still other varieties which prefer a combination of grain and a wood material, such as sawdust, or other cellulose based substrates. These latter types of substrates can be used to grow mushrooms directly from spawn without the need for compost.

In preparing the substrate for spawning, the integuments 10 can be automatically filled with substrate. A continuous sheet of integuments 10 may be continuously filled with substrate by an automated filling system similar to that disclosed in US Application Serial No 278,681, filed December 1, 1988, and entitled "Automated System for Micropropagation and Culturing Organic Materials", incorporated herein by reference. The sheet of integuments 10 have one side unsealed and open for the introduction of the substrate. A predetermined amount of substrate is introduced into each of the integuments by the automatic filling system. The integuments make it easier to automate the substrate filling operation because the continuous sheet of integuments can be disposed from a roll and automatically filled. It is more difficult to automate the filling of individual filter bags.

The integument and substrate are then sterilized. The substrate-filled integuments are introduced into an autoclave where they are heated to a temperature of 121.1°C (250°F) under a 10546 kg/m$^2$ (15 psi) pressure. This step can also be automated. The substrate-filled integuments are then removed from the autoclave and permitted to cool.

The open end of the substrate-filled integuments are then opened to receive mushroom culture to inoculate the substrate. The opened substrate-filled integuments can be inoculated by an automated inoculation system.

The automated inoculation system inoculates each substrate-filled integument with a predetermined amount of mushroom culture. The inoculated substrate-filled integument is closed capturing air within the integument and is then sealed by a heat sealer. The substrate is incubated, preferably within about one degree of 22.2°C (or two degrees of 72°F), after the substrate is inoculated. Capturing air within the integument at the time of sealing enhances the mixing of the substrate and inoculum. The captured air provides additional open space within the integument for mixing. To accomplish mixing, the integument is agitated while it is full of air.

The cultures grow in the substrate and form the thin thread-like cells which are the mushroom mycelium. After spawn for the Agaricus bisporus mushroom is produced, it can be mixed with compost. Since compost is not a completely sterile substrate, there is no need to complete the growth of the mushrooms in a sterile integument. Alternatively, the mushrooms can be grown directly on the spawn substrate, particularly for species other than Agaricus bisporus which do not require compost for growth.

In the production of oyster or straw mushrooms, the inoculated substrate-filled integument may be pasteurized, for example, by chemical, steam, or heat treatment. The sterilization and pasteurization of the substrate is advantageously carried out in the integument 10 of the invention. Thereafter, the substrate is inoculated and grown out in a sealed integument 10. Alternatively, the integument 10 could then be opened and the integument 10 could remain open during the oyster or straw mushroom growth phase.

If the mushroom is one which grows in a sealed environment, for example, the Cauliflower mushroom, the integument 10 can be kept sealed during the growth phase. In fact, it can be shipped while sealed so that it continues to grow during shipment. It can then be opened after arriving at the grower's location to complete the growing cycle. Alternatively, if it is a mushroom which grows in an open environment, the integument 10 can still remain sealed after inoculation and during shipment, so that the grower opens it to complete the growth cycle. Inasmuch as the integuments 10 are contaminant impermeable, mushroom species which heretofore could not be cultivated because of contamination problems during production, can now be produced.

The integuments 10 of the present invention are preferably used throughout the steps of spawning. As distinguished from growing the mycelium in conventional containers, i.e., glass bottles or plastic filter bags, it is advantageous to produce spawn in the integuments 10 because they allow gas exchange all around the outer surface of the spawn. It has been found that spawn will grow faster when placed in an integument 10 which is larger than a glass container, due to the enhanced gas exchange. Further the spawn will not desiccate, nor will there be non-uniform growth, as gas distribution around the spawn will be uniform. Further, unlike the problems associated with glass containers, there is no need for repackaging the spawn for shipment. Thus, labor is saved.

## Casing

Following mixing of the Agaricus bisporus spawn with the compost and the colonization of the compost, the compost is covered with a casing layer. As noted above, many species of mushrooms other than Agaricus bisporus do not require a casing layer. However, the straw mushroom, for example, produces at a faster rate if a casing layer is applied.

The casing layer's primary function is to induce sporophore production in quantity. The casing layer also serves to protect the compost from desiccation, pests and diseases, and to provide physical support for the developing mushroom fruit bodies.

The casing material can be soil, preferably a clay or clay-loam subsoil. Sphagnum peat mixtures can also be used. Casing is usually done two weeks after spawning, at which time the compost is well colonized.

The casing can be applied to the substrate after inoculation. The inoculated compost in the integument can be placed in an open container or an open integument 10 after casing.

It should also be noted that for other mushroom species, such as Shiitake or oyster mushrooms which do not require casing, the integuments 10 containing inoculated substrate can simply be cut open to serve as open containers for the mushroom culture. No casing needs to be applied. Because the spawn is not mixed with compost, the mushrooms are produced directly on the substrate on which spawn production takes place, e.g., on wood or a cellulose based substrate for oyster or Shiitake mushrooms.

## Pinning

After casing, the mycelium begin to grow rapidly and rhizomorphs form in the areas where the fine mycelium fuses together. Rhizomorphs, which appear as thick strings, form, sequentially, mushroom initials, primordia, and pins. Initials develop as outgrowths on the rhizomorphs after the rhizomorphs have formed on the casing. Once an initial becomes visible, the structure is a pin. Pins grow into buttons, which ultimately become the mushroom fruit bodies.

Pins develop when the carbon dioxide content of room air is lowered to less than 0.08 percent. This is done by introducing fresh air into the room. The relative humidity is kept high while the mycelium grows through the casing. For most species, pinning and fruit body development only takes place in the open environment, and cannot be done in a sealed integument.

Cropping

In the cropping phase, the mushrooms are harvested in 3 to 5 day cycles. Each cycle is followed by a dormancy period of about 7 to 10 days in which there is no production. The cycle repeats itself, and harvesting can go on as long as the mushrooms continue to mature.

Even if one is growing a mushroom which can withstand a sealed environment, e.g., the cauliflower mushroom, the integuments 10 are normally opened in the cropping phase in order to perform each harvesting cycle. As noted above, most types of mushrooms must be kept in an open environment throughout mushroom fruit body growth. For cauliflower mushrooms, it is preferable from a labor savings standpoint if the integuments 10 are opened only once at the start of the harvest cycle, and then not resealed after each cycle.

Alternatively, the contents of the integument 10 can be kept sealed from the atmosphere even during the cropping phase. This can be accomplished by pushing ripened mushroom fruit bodies away from the substrate and towards the end of the integument 10, and creating a new seal along the integument 10 between the portion of the integument 10 housing these ripened mushrooms and the portion housing the substrate so that the integument 10 now effectively includes two sections sealed from each other. The section of the integument 10 containing the ripened mushrooms is opened, and these mushrooms are removed. The newly formed seal prevents exposing the substrate and the growing culture to the atmosphere once the integument 10 is opened, thereby preventing contamination. This arrangement is desirable for those mushrooms which fruit in a sealed environment, for example, Cauliflower mushrooms.

Membrane Material 12

Polyethylene is the preferred material for membrane 12. Linear polyethylene is preferred over branched polyethylene. Linear polyethylene is distinguished from branched polyethylene by the method of manufacture in that linear polyethylene is polymerized in reactors maintained at pressures far lower than those required for branched polyethylene. Linear polyethylene encompasses ultra-low-density, linear low-density, high-density, high-molecular-weight high-density, and ultrahigh-molecular-weight polyethylene. Linear polyethylene density varies with the quantity of comonomer used with ethylene. The comonomer forms short-chain branches along the ethylene backbone. The greater the quantity of comonomer the lower the density of the polymer.

Polyethylene resins are characterized by their density. Linear low-density polyethylene has a density generally between 0.916 and 0.940 grams per cubic centimeter. High-density polyethylene is characterized by densities in the range of 0.940 to 0.965, with 0.962 grams per cubic centimeter as the practical upper limit.

Polyethylene resins are also characterized by a particular resin's molecular weight which is an inverse function of its melt index. Thus, melt index is a basic physical characteristic of a particular polyethylene resin. Most commercial high-density polyethylene resins have molecular weights in the range of 50,000 to 250,000. High-density polyethylenes are available with molecular weight distributions ranging from narrow to broad. Molecular weight distribution is generally expressed by the measurement of the resin's ability to flow under controlled conditions, i.e. its melt index. High-density polyethylene resins have melt indexes ranging from 0.04 to 75 grams per 10 minutes.

High-density polyethylene is preferred over low-density polyethylene for membrane 12 in the culturing and growing of living organic material. High-density polyethylene is a highly crystalline, lightweight thermoplastic. High-density polyethylene is semipermeable. Its principle characteristics are stiffness, good environmental stress-crack resistance, chemical resistance, toughness (even at low temperatures), dielectric properties, low-temperature properties, and relatively high softening temperature.

There are three basic manufacturing techniques for the production of high-density polyethylene: particle form slurry reactors, tandem reactors, and gas phase reactors. The particle form slurry reactor method is the most widely used and includes a supported transition metal catalyst slurried in a diluent that is circulated in a closed-loop reactor at a low pressure and low temperature. Ethylene, which is dissolved in the diluent, reacts with the catalyst to form solid high-density polyethylene particles. The particles are dried, combined with additives, and extruded as pellets.

High-density polyethylene can be processed by all melt forming methods, including extrusion, injection molding, rotational molding, blow molding, and powder coating. The primary property affecting processing is melt index. The largest amount of high-density polyethylene is consumed in blow molding applications. High-density polyethylene resins with a melt index in the 0.1 to 1.0 range generally are used. See Modern Plastics Encyclopedia, published by McGraw-Hill, Inc (1988) incorporated herein by reference.

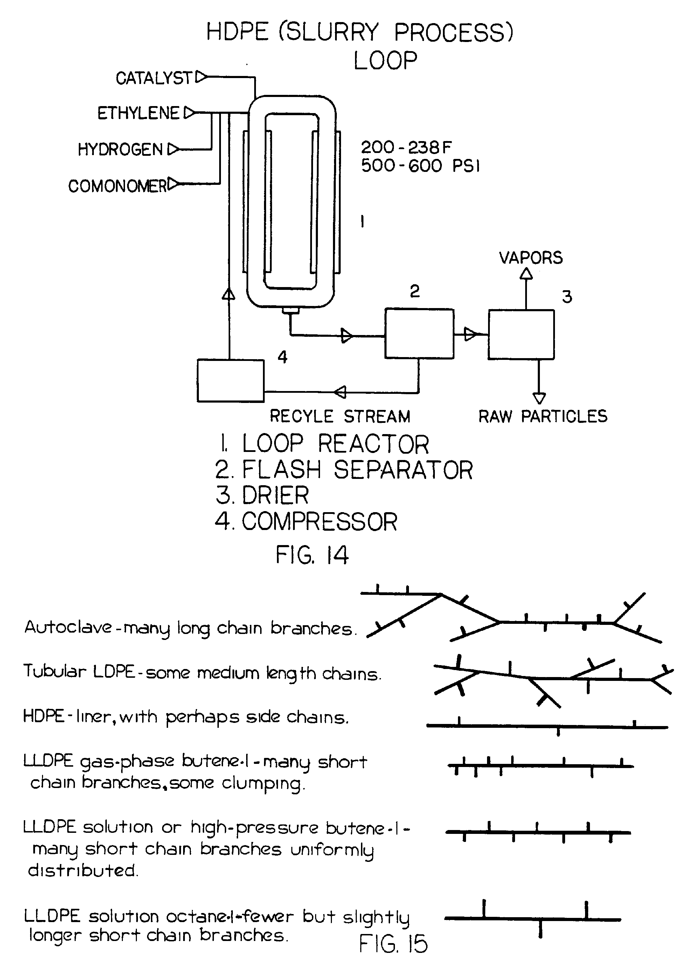

The high density polyethylene at a thickness of 1.25 mils forms a molecular structure during the extrusion process which is especially useful as membrane 12 for integuments 10, 50, 90. The high density polyethylene is made from linear crystalline polymers of suitable molecular weight with high tensile strength and extension modulus, a high degree of symmetry, strong intermolecular forces and a controlled degree of cross-linking between layers. The cross-links between adjacent layers of polymers are introduced to prevent the polymeric chains from slipping under applied stress. See Figure 15. The lightly cross-linked adjacent uniform layers of polymers of the high density polyethylene for membrane 12 form interstices therebetween which allow the preferred diffusion and osmosis therethrough for the desirable gas exchange between the ambient environment and the living organic material. These interstices are smaller than 0.01 micrometers so as to preclude the passage therethrough of even the smallest microorganisms, such as viruses. It also provides rigidity to facilitate the transfer and handling of the organic material.

For practising the invention described herein, it is preferred that the membrane material 12 have a permeability to $CO_2$ of from 200 to 1190 cc/645.16 cm$^2$ (100 sq in)/24 hours at 1.013 bar (1 atm) and a permeability to $O_2$ of from 100 to 424 cc/645.16 cm$_2$ (100 sq in)/24 hours at 1.013 bar (1 atm). Chevron HiD-9650, the preferred material for membrane 12, in the preferred 1.25 mil thickness, has a permeability of $CO_2$ of approximately 450 cc/645.16 cm$^2$ (100 sq in)/24 hours at 1.013 bar (1 atm) and a permeability to $O_2$ of approximately 190 cc/645.16 cm$^2$ (100 sq in)/24 hrs at 1.013 bar (1 atm).

Other materials which have the desired gas permeability and contaminant impermeability are also available for membrane 12. For example, certain low density polyethylene is suitable and even allows greater gas permeability than the preferred high density polyethylene; however, such low density polyethylene cannot withstand the high temperatures of the autoclave (121.1°C [250°F] at 1.013 bar [15 psi] for example) and the material melts or is otherwise deformed in the process. Accordingly, if such materials are used for membrane 12, they must be sterilized through other means.

The moisture vapour transmission rate (MVTR) is an important factor in the selection of materials for membrane 12. It is preferred that membrane 12 have a MVTR of from 0.2 to 0.684 gm/645.16 cm$^2$ (100 sq in)/24 hrs at 1.013 bar (1 atm). The preferred material, Chevron HiD-9650, in the preferred thickness of 1.25 mils, has a MVTR of 0.3 gm/645.16 cm$^2$ (100 sq in)/24 hours at 1.013 bar (1 atm). Membrane 12 of other materials, thicknesses, and permeabilities can be used depending upon the length of time the living organic material is to be grown in that integument. For example, the longer the growing period required for the particular organic material, the lower the MVTR of the membrane 12 should be so as to prevent the organic material from drying out to a degree that it will not be conducive to growth. Although other polymeric materials may have a greater permeability than the preferred high density polyethylene, if the permeability is too great, water within the integument 10 vaporizes and passes through membrane 12 and out of the integument.

The preferred high density polyethylene for membrane 12 is high-density polyethylene No HiD-9650, manufactured by the Chevron Chemical Company of Orange, Texas. HiD-9650 resin is a dry, fluffy powder as it exits the reaction process. This powder is run through a compounding extruder where it is melted and mixed with appropriate additives. As the 9650 polymer exits the compounding extruder, it is formed into round circular pellets which are subsequently packaged and shipped to customers for the manufacture of film through an extrusion process. Chevron has been manufacturing and selling Chevron HiD-9650 since approximately 1984. Its prior uses include the manufacture of notion and millinery bags and as barrier packaging.

As set forth in Chevron "Technical Data Sheet", CCO 9650-285 dated February 1986 and Chevron brochure "Chevron Polyethylene Resins for Extrusion", CCO 50001-885 dated August 1985, both incorporated herein by reference, HiD-9650 resin includes the following properties:

(a) Melt index of 0.3 grams per 10 minutes;
(b) Density of 0.950 grams per cubic centimetre;
(c) Nature colour; and
(d) Vicat softening point of 119.4°C (257°F).

HiD-9650 polyethylene film is manufactured from the HiD-9650 resin using the blown film extrusion process which is standard in the industry. HiD-9650 blown film having a nominal film thickness of 1.0 mils includes the following nominal properties:

(a) Spencer Impact of 3.2 inch-pounds per mil;
(b) 66 cm (26 inch) dart impact strength, F-50 of 90 grams per mil;
(c) Elmdorf tear, notched, MT/TD of 16/400 grams per mil;
(d) Tensile strength at break MD/TD of 5.2 x 10$^6$/4.4 x 10$^6$ kg/m$^2$ (7400/6300 psi);
(e) Elongation MD/TD of 460/650%;
(f) 1% Secant Modules MD/TD of 72.4 x 10$^6$/84.36 x 10$^6$ kg/m$^2$) (103,000/120,000 psi); and

(g) Moisture vapour transmission rate of 0.35 grams per 100 square inches per 24 hours per mil.

Chevron HiD-9650 is a high density, linear polyethylene copolymer resin made by the Phillip's loop reactor slurry process as depicted in Figure 14. Like all polyethylenes, Chevron HiD-9650 is a polymer of ethylene gas. To create Chevron HiD-9650, the ethylene gas can be reacted as a mixture with a comonomer, such as hexene or butene; Hydrogen as a polymerization terminator; and a catalyst initiator. The reaction results in a long chain, slightly branched molecule of ethylene and the comonomer as depicted in Figure 15. The density of HiD-9650 is achieved by the amount of comonomer that is reacted with the ethylene. The greater amount of the comonomer, the lower the density of the resulting polyethylene.

The recommended commercial processing conditions for converting HiD-9650 resin to a film with a nominal film thickness of 1.0 mil includes a melt temperature of 221.1°C (430°F) and a die gap of greater than 0.89 mm (0.035 inches). A smaller die gap may be used.

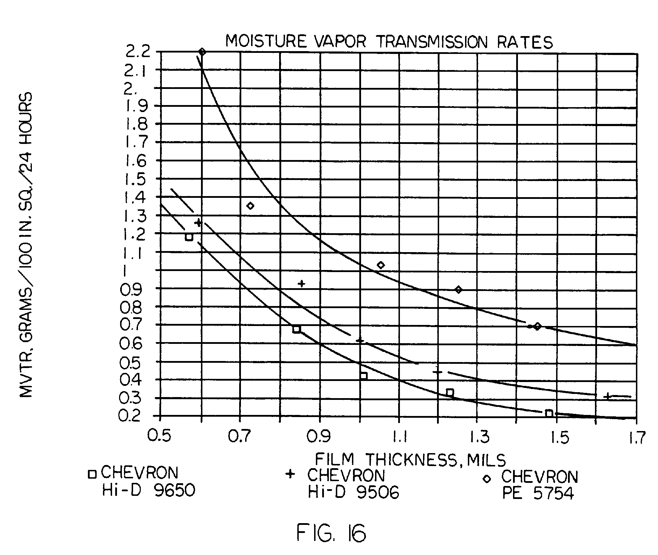

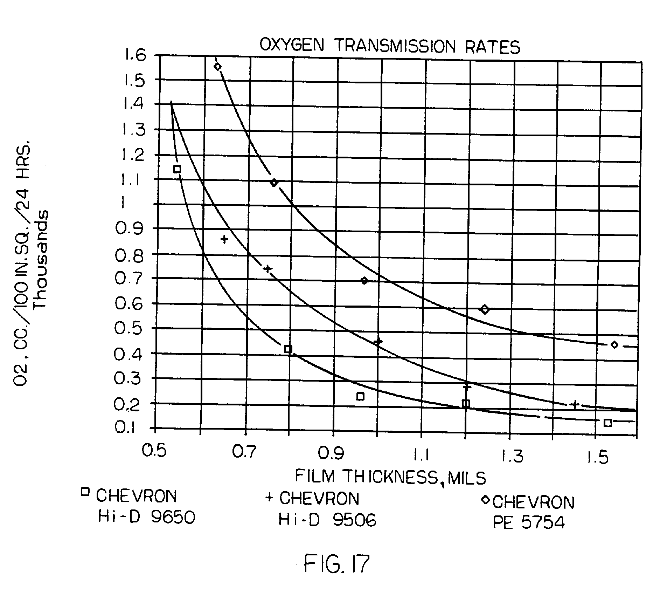

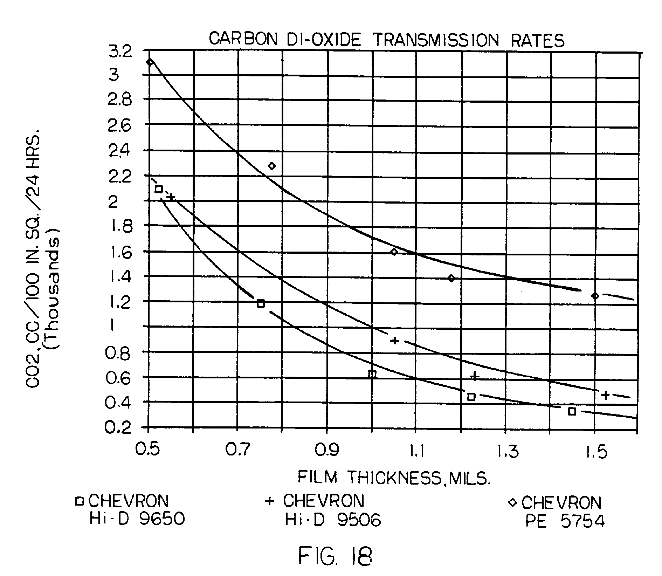

The gas transmission rates of moisture vapour, oxygen and carbon dioxide for various thicknesses of the preferred high density polyethylene (Chevron HiD-9650); another high density polyethylene (Chevron-9506); and a low density polyethylene (Chevron-PE5754) are set forth in the graphs of Figures 16, 17, and 18 and in tabular form below.

## GAS TRANSMISSION RATE TABLE

### SECTION I.  MOISTURE VAPOR (GRAMS/100 IN.SQ./24 HRS.)

| RESIN I.D. | Hi-D 9650 | | Hi-D 9506 | | PE 5754 | |
|---|---|---|---|---|---|---|
| TARGET FILM GAUGE, MILS. | MVTR | ACTUAL GAUGE | MVTR | ACTUAL GAUGE | MVTR | ACTUAL GAUGE |
| 0.50 | 1.190 | 0.570 | 1.261 | 0.590 | 2.200 | 0.600 |
| 0.75 | 0.684 | 0.840 | 0.932 | 0.850 | 1.358 | 0.720 |
| 1.00 | 0.432 | 1.010 | 0.623 | 1.000 | 1.035 | 1.050 |
| 1.20 | 0.339 | 1.230 | 0.455 | 1.200 | 0.897 | 1.250 |
| 1.50 | 0.223 | 1.480 | 0.320 | 1.630 | 0.700 | 1.450 |

### SECTION II. CARBON DIOXIDE (CC/100 IN.SQ./24 HRS.)

| RESIN I.D. | Hi-D 9650 | | Hi-D 9506 | | PE 5754 | |
|---|---|---|---|---|---|---|
| TARGET FILM GAUGE, MILS. | $CO_2TR$ | ACTUAL GAUGE | $CO_2TR$ | ACTUAL GAUGE | $CO_2TR$ | ACTUAL GAUGE |
| 0.50 | 2093.5 | 0.525 | 2031.5 | 0.550 | 3097.5 | 0.500 |
| 0.75 | 1190.5 | 0.750 | 1396.5 | 0.800 | 2281.0 | 0.775 |
| 1.00 | 620.0 | 1.000 | 886.0 | 1.050 | 1587.5 | 1.050 |
| 1.20 | 450.5 | 1.225 | 622.0 | 1.225 | 1407.5 | 1.175 |
| 1.50 | 339.0 | 1.450 | 463.5 | 1.525 | 1246.5 | 1.500 |

### SECTION III.  OXYGEN (CC/100 IN.SQ./24 HRS.)

| RESIN I.D. | Hi-D 9650 | | Hi-D 9506 | | PE 5754 | |
|---|---|---|---|---|---|---|
| TARGET FILM GAUGE, MILS. | $O_2TR$ | ACTUAL GAUGE | $O_2TR$ | ACTUAL GAUGE | $O_2TR$ | ACTUAL GAUGE |
| 0.50 | 1143.5 | 0.535 | 863.1 | 0.645 | 1556.5 | 0.625 |
| 0.75 | 424.4 | 0.795 | 746.0 | 0.745 | 1089.5 | 0.760 |
| 1.00 | 238.0 | 0.960 | 464.0 | 1.000 | 707.5 | 0.965 |
| 1.20 | 215.0 | 1.200 | 283.0 | 1.200 | 594.5 | 1.240 |
| 1.50 | 140.0 | 1.530 | 215.0 | 1.450 | 458.5 | 1.540 |

As can be seen, the thinner the membrane, the greater its transmission rate for these gases. In addition, it can be seen that for the three membrane materials tested, the low density material ( Chevron PE-5754) has the highest transmission rate for each of these gases. Although gas exchange is important to the growth of plants within integument 10, the preferred membrane 12 made from Chevron's HiD-9650, has a low moisture vapor transmission rate which is lower than the other materials tested. As noted above, if the moisture vapor transmission rates are too great, the media in the integument 10 will dry out too quickly and the organic material will desiccate and die, possibly before it can complete its growth.

The following table illustrates plant growth rates where various thicknesses and types of materials were used as membrane 12.

### PLANT GROWTH RATES

| MILS | DAYS | CHEVRON 9650 | CHEVRON 9506 | DUPONT 550 | CHEVRON 5754 |
|------|------|--------------|--------------|------------|--------------|
| 1.0 | | | | | |
| | 30 | 25.28 | 23.06 gms | 24.37 gms | ND |
| | | 27.58 | 23.61 | 24.98 | |
| | 60 | 33.42 | 24.27 | 23.21 | ND |
| | 90 | 36.01 | --- | 23.61 | ND |
| | 120 | 39.89 | --- | --- | ND |
| | 150 | 41.98 | --- | --- | ND |
| | 180 | 16.7 | 1.21 | 0.76 | ND |
| 1.25 | | | | | |
| | 30 | 25.46 | 24.61 | 24.62 | 24.52 gms |
| | 60 | 26.91 | 25.53 | 25.16 | 24.63 |
| | 90 | 32.41 | 25.91 | 25.92 | --- |
| | 120 | 36.06 | 26.01 | 26.23 | --- |
| | 150 | 39.21 | --- | 26.41 | --- |
| | 180 | 42.45 | --- | --- | --- |
| | | 16.99 | 1.4 | 1.79 | 0.11 |
| 1.5 | | | | | |
| | 30 | 25.31 | 24.31 | 24.69 | 24.56 |
| | 60 | 26.99 | 24.89 | 24.91 | 24.72 |
| | 90 | 33.21 | 25.21 | 25.13 | 24.81 |
| | 120 | 35.82 | 25.78 | 25.29 | --- |
| | 150 | 38.91 | --- | 25.51 | --- |
| | 180 | 41.98 | --- | --- | --- |
| | | 16.67 | 1.47 | 0.82 | 0.25 |

| mil | Days | | | | |
|---|---|---|---|---|---|
| 1.75 | | | | | |
| | 30 | 25.42 | 25.02 | 25.16 | ND |
| | 60 | 27.03 | 25.26 | 25.32 | ND |
| | 90 | 33.13 | 25.59 | 25.91 | ND |
| | 120 | 35.86 | 25.87 | 26.43 | ND |
| | 150 | 38.19 | 26.01 | 26.55 | ND |
| | 180 | 39.68 | --- | 26.88 | ND |
| | | 14.26 | 0.99 | 1.72 | |
| 2.0 | | | | | |
| | 30 | 25.36 | 25.31 | 25.28 | 24.98 |
| | 60 | 26.02 | 000 | 000 | 25.16 |
| | 90 | 26.49 | 000 | 000 | 25.36 |
| | 120 | 26.73 | 000 | 000 | 25.67 |
| | 150 | 000 | 000 | 000 | --- |
| | 180 | 000 | 000 | 000 | --- |
| | | 1.37 | | | 0.69 |
| 2.5 | | | | | |
| | 30 | 25.42 | 25.46 | 25.78 | 25.61 |
| | 60 | 25.78 | 000 | 000 | 25.92 |
| | 90 | 25.96 | 000 | 000 | 26.21 |
| | 120 | 000 | 000 | 000 | 26.38 |
| | 150 | 000 | 000 | 000 | --- |
| | 180 | 000 | 000 | 000 | --- |
| | | 0.54 | | | 0.77 |

ND  – MEMBRANE MATERIAL NOT AVAILABLE (NO DATA)

--  – PLANT MATERIAL DIED DUE TO DESICCATION

000  – NO CHANGE FROM PREVIOUS RECORDING

As shown in the above table, growth rates were recorded when membranes of high density polyethylene (Chevron HiD-9650, Chevron-9506, Dupont-550) and low density polyethylene (Chevron-PE-5754) were used in an integument pack 50. To conduct this test, fifty integument packs 50 were obtained in each mil thickness shown in the table for each of the four membrane materials (excluding 1.0 mil and 1.75 mil thicknesses of Chevron-PE-5754 which were not available). Under a laminar flow hood, equal amounts of media were placed in the cellules of each sterilized integument pack 50. Each cellule was then invested with a sample of plant tissue and the integument pack 50 was then sealed to prevent the introduction of contaminants. Integument packs 50 were then placed in the culture area of the laboratory. At thirty day intervals, each integument pack 50 was weighed, and an average weight of an integument was calculated for each of the fifty samples of the same membrane material and the same mil thickness. This data was recorded and is reproduced in the above table. After one hundred eighty days of the experiment, the thirty day average weight was subtracted from the last recorded weight and the difference was recorded in the above table below the one hundred eighty day weight. As can be seen, the preferred membrane 12 of Chevron high density polyethylene HiD-9650 enabled plant material to grow at greater rates and for longer periods of time than the other materials tested.

The above examples demonstrate that the integument and method of the present invention yields dramatic improvements in the culturing and growing of living organic material. These same improvements will follow irrespective of whether the plants cultured are horticultural, agricultural or even aquatic in variety. It is believed that the invention will yield dramatic improvements in animal and human tissue culturing as well. The integument and method described herein may be used for culturing microorganisms, such as viruses, single celled algae, fungus and bacteria, and in preventing the cultured microorganisms from escaping from the integument and contaminating or infecting others.

The term "mil" (used throughout the above description) is 0.001 inch or 0.025 mm. Also "foot-candle" (used above) is equivalent to 10.764 lux, 100 square inches is equivalent to 645.16 square centimetres, and 1 inch-pound is equivalent to 1.15 cm-kg.

**Claims**

1. A system for the culturing of organic material, comprising:

   a medium for growing the organic material;

   a polyethylene membrane forming a chamber for enclosing the organic material and completely sealing the organic material and medium within said chamber; said polyethylene membrane being gas permeable and liquid impermeable and having interstices smaller than 0.01 micrometers, thereby allowing gas exchange through said membrane for the culturing of the organic material and preventing the exposure of the organic material to viral biological contaminants.

2. A system as claimed in claim 1, wherein said chamber has a non-artificial internal and external pressure, the gas mixture and pressure of the atmospheric ambient environment being permitted to equalize by gas exchange through said membrane.

3. A system as claimed in claim 1, wherein said membrane is made of high-density polyethylene.

4. A system as claimed in claim 1, wherein said membrane has a moisture vapour transmission rate equal to that of a high-density polyethylene film with a thickness in the range of 0.025 - 0.05 mm (0.001 - 0.002 inch).

5. A system as claimed in claim 1, wherein said membrane is translucent to permit light to impinge on the organic material.

6. A system as claimed in claim 1, wherein said chamber is free of an internal containment device.

7. A system as claimed in claim 1, wherein said membrane transmits therethrough light rays having a wave length between 400 and 750 nanometres.

8. A system as claimed in claim 1, wherein said membrane is made of a single layer of high-density polyethylene.

9. A system as claimed in claim 1, wherein said membrane allows the transmission of light therethrough except for any light blocked by the organic material.

10. A system as claimed in claim 1, wherein the moisture vapour transmission is less than 0.68 grams per 645.16 $cm^2$ (100 square inches) per 24 hours at 1.013 bar (1 atmosphere).

11. A system as claimed in claim 1, wherein said membrane will withstand a temperature of 121.1°C (250°F) at a pressure of 10546 $kg/m^2$ (15 psi) without deformation of said membrane.

12. A system as claimed in claim 1, wherein said membrane has a carbon dioxide transmission rate between 200 and 1190 cubic centimetres per 645.16 $cm^2$ (100 square inches) per 24 hours at 1.013 bar (1 atmosphere).

13. A system as claimed in claim 1, wherein said membrane has an oxygen transmission rate between 100 and 424 cubic centimetres per 645.16 $cm^2$ (100 square inches) per 24 hours at 1.013 bar (1 atmosphere).

14. A system as claimed in claim 1, wherein said membrane has a moisture vapour transmission rate of between 0.2 and 0.68 grams per 645.16 $cm^2$ (100 square inches) per 24 hours at 1.013 bar (1 atmosphere).

15. A system as claimed in claim 1, wherein said membrane is made of Chevron high-density polyethylene 9650.

16. A method for growing organic material, comprising the steps of:

   inserting the organic material and a growth medium in the cellule of a container made of a gas permeable, liquid and contaminant impermeable membrane having interstices smaller than 0.01 micrometers;

   enclosing completely the organic material within the cellule; and

   completely sealing the organic material from viral biological contaminants in the ambient environment.

17. A method as claimed in claim 16, wherein the growing organic material is plant tissue.

18. A method as claimed in claim 16, wherein the growing organic material is mammalian tissue.

19. A method as claimed in claim 16, wherein the growing organic material is a plant.

20. A method as claimed in claim 16, wherein the growing organic material is a seed.

21. A method as claimed in claim 16, wherein the growing organic material is bacteria.

22. A method as claimed in claim 16, wherein the growing organic material is a microorganism.

23. A method as claimed in claim 16, wherein the growing organic material is a fungus.

24. A method as claimed in claim 16, wherein the growing organic material is an algae.

25. A method as claimed in claim 16, further including the step of transmitting oxygen through the membrane at a rate between 100 and 424 cubic centimetres per 645.16 $cm^2$ (100 square inches) per 24 hours at 1.013 bar (1 atmosphere).

26. A method as claimed in claim 16, further including the step of transmitting carbon dioxide through the membrane at a rate between 200 and 1190 cubic centimetres per 645.16 $cm^2$ (100 square inches) per 24 hours at 1.013 bar (1 atmosphere).

27. A method as claimed in claim 16, further including the step of transmitting moisture vapour through the membrane at a rate no greater than 0.68 grams per 645.16 $cm^2$ (100 square inches) per 24 hours at 1.013 bar (1 atmosphere).

28. A method as claimed in claim 16, wherein the organic material is a seed for growing in the atmospheric ambient environment, comprising the steps of:
    (a) transferring an organic medium to a cellule made of a gas permeable, liquid impermeable and translucent membrane;
    (b) surface sterilizing the seed;
    (c) transferring the seed to the cellule;
    (d) enclosing the seed within the cellule to protect the seed from biological contaminants in the ambient environment;
    (e) exposing all parts of the seed to the light transmitted and gas exchanged through the cellule; and
    (f) removing the plantlet from the cellule upon development of foliage and a root system.

**Patentansprüche**

1. System zur Züchtung von organischem Material, bestehend aus:
    einem Nährmedium für das organische Material und
    einer Polyethylenmembran, die eine das organische Material einhüllende Kammer bildet, wobei das organische Material und das Nährmedium in besagter Kammer vollständig eingeschlossen sind und wobei besagte Polyethylenmembran gasdurchlässig und flüssigkeitsundurchlässig ist und Zwischenräume mit einer Größe von unter 0,01 Mikrometer besitzt, wodurch Gasaustausch durch besagte Membran zur Züchtung des organischen Materials ermöglicht wird und ein Kontakt zwischen dem organischen Material und biologischen Verunreinigungen in der Form von Viren verhindert wird.

2. System gemäß Anspruch 1, dadurch gekennzeichnet, daß besagte Kammer einen nicht künstlichen inneren und äußeren Druck aufweist, wobei ein Ausgleich der Gasmischung und des Drucks der Umgebungsatmosphäre mittels Gasaustausch durch besagte Membran ermöglicht wird.

3. System gemäß Anspruch 1, dadurch gekennzeichnet, daß besagte Membran aus Polyethylen hoher Dichte besteht.

4. System gemäß Anspruch 1, dadurch gekennzeichnet, daß besagte Membran die gleiche Wasser dampfdurchlässigkeit besitzt wie eine Schicht aus Polyethylen hoher Dichte mit einer Dicke im Bereich von

EP 0 418 323 B1

0,025-0,05 mm (0,001-0,002 Zoll).

5. System gemäß Anspruch 1, dadurch gekennzeichnet, daß besagte Membran lichtdurchlässig ist, um den Einfall von Licht auf das organische Material zu ermöglichen.

6. System gemäß Anspruch 1, dadurch gekennzeichnet, daß besagte Kammer keine innere Einschlußeinrichtung enthält.

7. System gemäß Anspruch 1, dadurch gekennzeichnet, daß besagte Membran für Lichtstrahlen mit einer Wellenlänge zwischen 400 und 750 Nanometer durchlässig ist.

8. System gemäß Anspruch 1, dadurch gekennzeichnet, daß besagte Membran aus einer einzigen Schicht aus Polyethylen hoher Dichte besteht.

9. System gemäß Anspruch 1, dadurch gekennzeichnet, daß besagte Membran für Licht, ausgenommen für nicht vom organischen Material durchgelassenes Licht, durchlässig ist.

10. System gemäß Anspruch 1, dadurch gekennzeichnet, daß die Wasserdampfdurchlässigkeit unter 0,68 Gramm pro 645,16 cm² (100 Quadratzoll) und 24 Stunden bei 1,013 Bar (1 Atmosphäre) beträgt.

11. System gemäß Anspruch 1, dadurch gekennzeichnet, daß besagte Membran bei einer Temperatur von 121,1°C (250°F) bei einem Druck von 10546 kg/m² (15 psi) Bestand hat, ohne daß eine Verformung besagter Membran eintritt.

12. System gemäß Anspruch 1, dadurch gekennzeichnet, daß die Kohlendioxiddurchlässigkeit besagter Membran zwischen 200 und 1190 Kubikzentimeter pro 645,16 cm² (100 Quadratzoll) und 24 Stunden bei 1,013 Bar (1 Atmosphäre) beträgt.

13. System gemäß Anspruch 1, dadurch gekennzeichnet, daß die Sauerstoffdurchlässigkeit besagter Membran zwischen 100 und 424 Kubikzentimeter pro 645,16 cm² (100 Quadratzoll) und 24 Stunden bei 1,013 Bar (1 Atmosphäre) beträgt.

14. System gemäß Anspruch 1, dadurch gekennzeichnet, daß die Wasserdampfdurchlässigkeit besagter Membran zwischen 0,2 und 0,68 Gramm pro 645,16 cm² (100 Quadratzoll) und 24 Stunden bei 1,013 Bar (1 Atmosphäre) beträgt.

15. System gemäß Anspruch 1, dadurch gekennzeichnet, daß besagte Membran aus Polyethylen hoher Dichte von Typ Chevron 9650 besteht.

16. Verfahren zur Züchtung von organischem Material, dadurch gekennzeichnet, daß es folgende Schritte umfaßt:
Einfügen des organischen Materials und eines Wachstumsmediums in die Zelle eines Behälters, der aus einer gasdurchlässigen und flüssigkeitssowie verunreinigungsundurchlässigen Membran mit Zwischenräumen mit einer Größe von unter 0,01 Mikrometer besteht;
vollständiges Einhüllen des organischen Materials in der Zelle; und
vollständiges Absiegeln des organischen Materials gegen biologische Verunreinigungen in der Umgebung in der Form von Viren.

17. Verfahren gemäß Anspruch 16, dadurch gekennzeichnet, daß es sich bei dem wachsenden organischen Material um pflanzliches Gewebe handelt.

18. Verfahren gemäß Anspruch 16, dadurch gekennzeichnet, daß es sich bei dem wachsenden organischen Material um Säugetiergewebe handelt.

19. Verfahren gemäß Anspruch 16, dadurch gekennzeichnet, daß es sich bei dem wachsenden organischen Material um eine Pflanze handelt.

20. Verfahren gemäß Anspruch 16, dadurch gekennzeichnet, daß es sich bei dem wachsenden organischen Material um einen Samen handelt.

21. Verfahren gemäß Anspruch 16, dadurch gekennzeichnet, daß es sich bei dem wachsenden organischen

21

Material um Bakterien handelt.

22. Verfahren gemäß Anspruch 16, dadurch gekennzeichnet, daß es sich bei dem wachsenden organischen Material um einen Mikroorganismus handelt.

23. Verfahren gemäß Anspruch 16, dadurch gekennzeichnet, daß es sich bei dem wachsenden organischen Material um einen Pilz handelt.

24. Verfahren gemäß Anspruch 16, dadurch gekennzeichnet, daß es sich bei dem wachsenden organischen Material um eine Alge handelt.

25. Verfahren gemäß Anspruch 16, dadurch gekennzeichnet, daß es weiterhin einen Schritt umfaßt, in dem zwischen 100 und 424 Kubikzentimeter Sauerstoff pro 645,16 cm² (100 Quadratzoll) und 24 Stunden bei 1,013 Bar (1 Atmosphäre) durch die Membran durchgelassen werden.

26. Verfahren gemäß Anspruch 16, dadurch gekennzeichnet, daß es weiterhin einen Schritt umfaßt, in dem zwischen 200 und 1190 Kubikzentimeter Kohlendioxid pro 645,16 cm² (100 Quadratzoll) und 24 Stunden bei 1,013 Bar (1 Atmosphäre) durch die Membran durchgelassen werden.

27. Verfahren gemäß Anspruch 16, dadurch gekennzeichnet, daß es weiterhin einen Schritt umfaßt, in dem nicht mehr als 0,68 Gramm Wasserdampf pro 645,16 cm² (100 Quadratzoll) und 24 Stunden bei 1,013 Bar (1 Atmosphäre) durch die Membran durchgelassen werden.

28. Verfahren gemäß Anspruch 16, dadurch gekennzeichnet, daß es sich bei dem organischen Material um einen Samen handelt, der in der Umgebungsatmosphäre wachsen soll, wobei das Verfahren folgende Schritte umfaßt:
(a) Einfügen eines organischen Nährmediums in eine aus einer gasdurchlässigen, flüssigkeitsundurchlässigen, lichtdurchlässigen Membran bestehende Zelle;
(b) Oberflächensterilisierung des Samens;
(c) Einsetzen des Samens in die Zelle;
(d) Einhüllen des Samens in der Zelle, um den Samen vor biologischen Verunreinigungen in der Umgebung zu schützen;
(e) Aussetzen aller Samenteile dem durch die Zelle durchgelassenen Licht und dem durch die Zelle ausgetauschten Gas; und
(f) Entfernen des Pflänzchens aus der Zelle, sobald sich Blattapparat und Wurzelsystem gebildet haben.

## Revendications

1. Système de culture d'une matière organique, comprenant :
un milieu de culture de la matière organique ;
une membrane de polyéthylène formant une chambre renfermant la matière organique et isolant complètement la matière organique et le milieu dans ladite chambre ; ladite membrane de polyéthylène étant perméable aux gaz et imperméable aux liquides, et ayant des interstices inférieurs à 0,01 micromètre, permettant ainsi un échange à travers ladite membrane pour effectuer la culture de la matière organique et éviter d'exposer la matière organique à des contaminants biologiques viraux.

2. Système selon la revendication 1, dans lequel ladite chambre présente une pression interne et externe naturelle, la pression du mélange gazeux et celle de l'atmosphère ambiante pouvant s'équilibrer par échange à travers ladite membrane.

3. Système selon la revendication 1, dans lequel ladite membrane est en polyéthylène haute densité.

4. Système selon la revendication 1, dans lequel ladite membrane présente un taux de transmission de la vapeur d'eau égal à celui d'un film de polyéthylène haute densité ayant une épaisseur dans la gamme de 0,025-0,05 mm (0,001 -0,002 pouce).

5. Système selon la revendication 1, dans lequel ladite membrane est translucide, pour permettre l'incidence de la lumière sur la matière organique.

**6.** Système selon la revendication 1, dans lequel ladite chambre ne comporte pas d'enceinte de confinement interne.

**7.** Système selon la revendication 1, dans lequel ladite membrane permet la transmission de rayons lumineux ayant une longueur d'onde comprise entre 400 et 750 nanomètres.

**8.** Système selon la revendication 1, dans lequel ladite membrane est constituée d'une monocouche de polyéthylène haute densité.

**9.** Système selon la revendication 1, dans lequel ladite membrane permet la transmission de la lumière, sauf celle de la lumière arrêtée par la matière organique.

**10.** Système selon la revendication 1, dans lequel la transmission de la vapeur d'eau est inférieure à 0,68 g par 645, 16 cm$^2$ (100 pouces$^2$) par 24 heures à 1,013 bar (1 atmosphère).

**11.** Système selon la revendication 1, dans lequel ladite membrane résiste à une température de 121,1°C (250°F) à une pression de 10546 kg/m$^2$ (15 psi), sans déformation de ladite membrane.

**12.** Système selon la revendication 1, dans lequel ladite membrane présente un taux de transmission du dioxyde de carbone compris entre 200 et 1190 cm$^3$ par 645,16 cm$^2$ (100 pouces$^2$) par 24 heures à 1,013 bar (1 atmosphère).

**13.** Système selon la revendication 1, dans lequel ladite membrane présente un taux de transmission de l'oxygène compris entre 100 et 424 cm$^3$ par 645,16 cm$^2$ (100 pouces$^2$) par 24 heures à 1,013 bar (1 atmosphère).

**14.** Système selon la revendication 1, dans lequel ladite membrane présente un taux de transmission de la vapeur d'eau compris entre 0,2 et 0,68 g par 645,16 cm$^2$ (100 pouces$^2$) par 24 heures à 1,013 bar (1 atmosphère).

**15.** Système selon la revendication 1, dans lequel ladite membrane est en polyéthylène haute densité 9650 de Chevron.

**16.** Procédé de culture de matière organique, comprenant les étapes consistant à :
insérer la matière organique et un milieu de culture dans la cellule d'un conteneur constitué d'une membrane perméable aux gaz, imperméable aux liquides et aux contaminants, ayant des interstices inférieurs à 0,01 micromètre ;
inclure complètement la matière organique dans la cellule ; et
isoler complètement la matière organique des contaminants biologiques viraux de l'environnement.

**17.** Procédé selon la revendication 16, dans lequel ladite matière organique en culture est un tissu végétal.

**18.** Procédé selon la revendication 16, dans lequel ladite matière organique en culture est un tissu de mammifère.

**19.** Procédé selon la revendication 16, dans lequel ladite matière organique en culture est une plante.

**20.** Procédé selon la revendication 16, dans lequel ladite matière organique en culture est une semence.

**21.** Procédé selon la revendication 16, dans lequel ladite matière organique en culture est une bactérie.

**22.** Procédé selon la revendication 16, dans lequel ladite matière organique en culture est un microorganisme.

**23.** Procédé selon la revendication 16, dans lequel ladite matière organique en culture est un champignon.

**24.** Procédé selon la revendication 16, dans lequel ladite matière organique en culture est une algue.

**25.** Procédé selon la revendication 16, comprenant en outre l'étape consistant à transmettre l'oxygène à travers la membrane à un taux compris entre 100 et 424 cm$^3$ par 645,16 cm$^2$ (100 pouces$^2$) par 24 heures à 1,013 bar (1 atmosphère).

26. Procédé selon la revendication 16, comprenant en outre l'étape consistant à transmettre le dioxyde de carbone à travers la membrane à un taux compris entre 200 et 1190 cm$^3$ par 645,16 cm$^2$ (100 pouces$^2$) par 24 heures à 1,013 bar (1 atmosphère).

27. Procédé selon la revendication 16, comprenant en outre l'étape consistant à transmettre la vapeur d'eau à travers la membrane à un taux ne dépassant pas 0,68 g par 645,16 cm$^2$ (100 pouces$^2$) par 24 heures à 1,013 bar (1 atmosphère).

28. Procédé selon la revendication 16, dans lequel la matière organique est ensemencée pour effectuer la culture dans l'atmosphère environnante, comprenant les étapes consistant à :
(a) transférer un milieu organique dans une cellule constituée d'une membrane perméable aux gaz, imperméable aux liquides et translucide ;
(b) stériliser en surface le germe;
(c) transférer le germe dans la cellule ;
(d) inclure le germe dans la cellule pour protéger le germe des contaminants biologiques du milieu environnant ;
(e) exposer toutes les parties du germe à la lumière transmise et au gaz échangé à travers la cellule ;
(f) enlever la plantule de la cellule après développement du système foliaire et des racines.

FIG. 1

FIG. 2

FIG. 4

FIG. 5

V FIG. 7

FIG. 6

FIG. 8

FIG. 3

FIG. 9

FIG. 10

FIG. 11

FIG. 12

FIG. 13

HDPE (SLURRY PROCESS)
LOOP

CATALYST ▷
ETHYLENE ▷
HYDROGEN ▷
COMONOMER ▷

200 - 238 F
500 - 600 PSI

VAPORS

RECYLE STREAM    RAW PARTICLES

1. LOOP REACTOR
2. FLASH SEPARATOR
3. DRIER
4. COMPRESSOR

FIG. 14

Autoclave - many long chain branches.

Tubular LDPE - some medium length chains.

HDPE - liner, with perhaps side chains.

LLDPE gas-phase butene-1 - many short
chain branches, some clumping.

LLDPE solution or high-pressure butene-1 -
many short chain branches uniformly
distributed.

LLDPE solution octane-1-fewer but slightly
longer short chain branches.     FIG. 15

FIG. 16

FIG. 17

CARBON DI-OXIDE TRANSMISSION RATES

FIG. 18